# EUROPÄISCHE PATENTSCHRIFT

(11) **EP 1 123 293 B1**
(45) Veröffentlichungstag und Bekanntmachung des Hinweises auf die Patenterteilung: **03.08.2005**
(21) Anmeldenummer: 99950699.1
(22) Anmeldetag: 11.10.1999
(51) Int. Cl.: C07D 471/22, C09B 5/62

(54) **KERNERWEITERTE PERYLENBISIMIDE UND IHRE VERWENDUNG ALS FLUORESZENZFARBSTOFFE**
CORE-EXTENDED PERYLENE BISIMIDES
BISIMIDES DE PERYLENE A NOYAU ELARGI ET LEUR UTILISATION COMME COLORANTS FLUORESCENTS

(30) Priorität: 21.10.1998 DE 19848555
(43) Veröffentlichungstag der Anmeldung: 16.08.2001
(73) Patentinhaber: Ciba Specialty Chemicals Holding Inc., 4057 Basel (CH)
(72) Erfinder: LANGHALS, Heinz, D-85521 Ottobrunn (DE); KIRNER, Susanne, 69207 Sandhausen (DE); BLANKE, Patrick, D-82229 Seefeld (DE); SPECKBACHER, Markus, D-84562 Mettenheim-Hart (DE)
(86) Internationale Anmeldenummer: PCT/EP1999/007614
(87) Internationale Veröffentlichungsnummer: WO 2000/023446

(56) Entgegenhaltungen:
- EP-A- 0 039 085
- EP-A- 0 638 613
- EP-A- 0 896 964
- US-A- 4 937 164
- US-A- 5 645 965
- LANGHALS H: "CYCLIC CARBOXYLIC IMIDE STRUCTURES AS STRUCTURE ELEMENTS OF HIGH STABILITY. NOVEL DEVELOPMENTS IN PERYLENE DYE CHEMISTRY" HETEROCYCLES,NL,ELSEVIER SCIENCE PUBLISHERS B.V. AMSTERDAM, Bd. 40, Nr. 1, 1. Januar 1995 (1995-01-01), Seiten 477-500, XP000653314 ISSN: 0385-5414 in der Anmeldung erwähnt
- SEYBOLD G ET AL: "NEW PERYLENE AND VIOLANTHRONE DYESTUFFS FOR FLUORESCENT COLLECTORS" DYES AND PIGMENTS,GB,ELSEVIER APPLIED SCIENCE PUBLISHERS. BARKING, Bd. 11, Nr. 4, 1. Januar 1989 (1989-01-01), Seiten 303-317, XP000084462 ISSN: 0143-7208
- ZANDER,M.: "Reaktionen mehrkerniger aromatischer Kohlenwasserstoffe mit Phenyl-1,2,4-triazolin-3,5-dion" CHEMIKER-ZEITUNG, Bd. 99, Nr. 2, 1975, Seiten 92-93, XP000872276 HEIDELBERG in der Anmeldung erwähnt
- SOLOMENTSEVA,T.I. ET AL.: "Chemistry of Perylene. High-temperature Sulfurisation of BAY-substituted Perylene-3,4,9,10-Tetracarboxylic Acids" J.ORG.CHEM.USSR, Bd. 22, 1986, Seiten 943-946, XP000872255 MOSCOW in der Anmeldung erwähnt
- DEMMIG,S. ET AL.: "Leichtlösliche, lichtechte Perylen-Fluoreszenzfarbstoffe" CHEM.BER., Bd. 121, 1988, Seiten 225-2230, XP000881317 WEINHEIM
- QUANTE H ET AL: "SYNTHESIS OF SOLUBLE PERYLENEBISAMIDINE DERIVATIVES. NOVEL LONG-WAVELENGTH ABSORBING AND FLUORESCENT DYES" CHEMISTRY OF MATERIALS,US,AMERICAN CHEMICAL SOCIETY, WASHINGTON, Bd. 9, Nr. 2, 1. Februar 1997 (1997-02-01), Seiten 495-500, XP000671729 ISSN: 0897-4756
- KAISER ET AL: "Synthese von nichtsymmetrisch substituierten Perylen-Fluoreszenzfarbstoffen" CHEMISCHE BERICHTE,DE,VERLAG CHEMIE GMBH. WEINHEIM, Bd. 124, Nr. 3, 1991, Seiten 529-535-535, XP002110435 ISSN: 0009-2940
- NAGAO,Y. ET AL.: "Synthesis and Properteis of N-alkyl-N'-aryl--3,4:9,10-Perylenebis(dica rboximide)" DYES AND PIGMENTS, Bd. 5, 1984, Seiten 171-188, XP000881349 BARKING,ESSEX
- NAGAO,Y. ET AL.: "Synthesis of unsymetrical Perylenebis(dicarboximide) derivatives " CHEMISTRY LETTERS, - 1979 Seiten 151-154, XP000881254 TOKYO
- GEERTS Y ET AL: "QUATERRYLENEBIS(DICARBOXIMIDE)S: NEAR INFRARED ABSORBING AND EMITTING DYES" JOURNAL OF MATERIALS CHEMISTRY,GB,THE ROYAL SOCIETY OF CHEMISTRY, CAMBRIDGE, Bd. 8, Nr. 11, November 1998 (1998-11), Seiten 2357-2369, XP000803180 ISSN: 0959-9428

## Beschreibung

Die vorliegende Erfindung betrifft kernerweiterte Perylenbisimide der allgemeinen Formeln
(I) und (II) 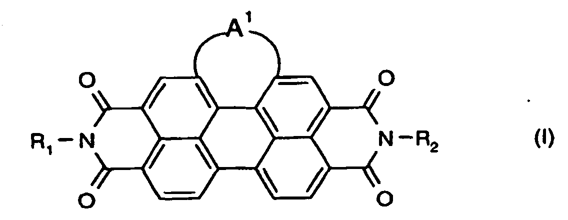 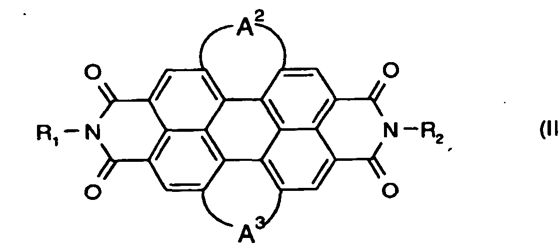
worin
R¹ und R² unabhängig voneinander für 1-(C₁-C₉Alkyl)-C₂-C₁₀Alkyl, C₃-C₂₄Cycloalkyl, oder C₆-C₁₀Aryl stehen, und
A¹ und A³ unabhängig voneinander für -CH=CH-, oder R³OOC-C(-)=C(-)-COOR³, stehen, und
A² für eine Verbindung der Formeln (III), (IV) oder (V) 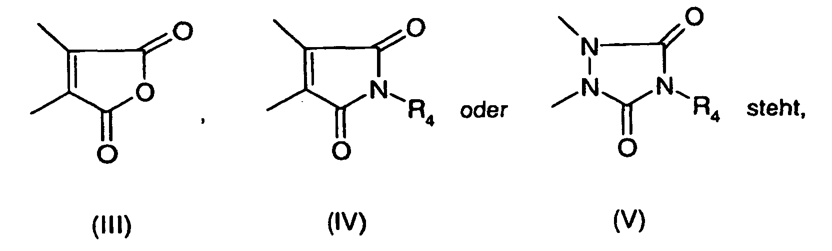 worin
R³ für Wasserstoff, C₁-C₂₄Alkyl oder C₃-C₂₄Cycloalkyl und
R⁴ für unsubstituiertes oder substituiertes C₁-C₂₄Alkyl, C₃-C₂₄Cycloalkyl, Phenyl, Benzyl, -CO-C₁-C₄Alkyl, -CO-C₆H₅ oder C₁-C₄Alkylcarbonsäure-(C₁-C₄Alkyl)-Ester stehen,
Verfahren zur Herstellung der Verbindungen (I) und (II) sowie deren Verwendung als Farbmittel.

Perylene sind bekanntermassen (siehe Heterocycles, Vol. 40, No. 1, (1995) 477-500) photostabile Fluoreszenzfarbstoffe, die sich häufig durch hohe Fluoreszenzquantenausbeuten auszeichnen. Nachteilig ist allerdings, dass Perylene im wässrigen Medien wenig löslich sind.

In Chem. Ztg. (1975), 99, 92-93 werden unter anderem durch Diels-Alder-Reaktion von 4-Phenyl-1,2,4-triazolin-3,5-dion mit Perylen zugängliche kernerweiterte Perylene beschrieben, die Absorptionen bei 600 nm aufweisen können.

Weitere Diels-Alder-Reaktionen an Perylenen sind in Heterocycles, Vol. 40, No. 1, 1995 beschrieben. In J. Org. Chem. USSR, (1986), 22, 943-946 ist die Herstellung von Thio- und Dithio-cyclischen Derivaten der Perylen-3,4,9,10-tetracarbonsäure beschrieben. Des weiteren ist in J. Org. Chem. USSR, (1980), 16, 762-7 ein Verfahren zur Herstellung von Stickstoffcyclischen Derivaten der Perylen-3,4,9,10-tetracarbonsäure beschrieben.

US-A-4,937,164 offenbart spezifische Perylenbisimide-Derivate mit einem charakteristischen Strukturelement der Formel -S- und -S-S- .

Der Erfindung lag daher die Aufgabe zugrunde, weitere kernerweiterte Perylene, insbesondere kernerweiterte Perylenbisimide bereitzustellen, die bevorzugt als Fluoreszenzfarbmittel gute Hitze- und Lichtechtheit besitzen, des weiteren sollten Perylene, die sich als NIR-Farbstoffe oder Fluoreszenzmarker eignen, zur Verfügung gestellt werden.

Demgemäss wurden die eingangs definierten kernerweiterten Perylenbisimide der allgemeinen Formeln (I) und (II) gefunden.

C₁-C₂₄-Alkyl bedeutet beispielsweise Methyl, Ethyl, n-, i-Propyl, n-, i-, sek.-, tert.-Butyl, n-, neo-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, Heneicosyl, Docosyl oder Tetracosyl, vorzugsweise 1-(C₁-C₉Alkyl)-C₂-C₁₀Alkyl wie 1-Methyl-ethyl, 1-Ethyl-n-propyl, 1-n-Propyl-n-butyl, 1-n-Butyl-n-pentyl, 1-n-Hexyl-1-n-heptyl, 1-n-Heptyl-1-n-octyl, 1-n-Octyl-1-n-nonyl, 1-n-Nonyl-1-n-decyl, oder C₁-C₈-Alkyl wie Methyl, Ethyl, n-, i-Propyl, n-, i-, sek.-, tert.-Butyl, n-, neo-Pentyl, n-Hexyl, n-Heptyl oder n-Octyl und besonders bevorzugt C₁-C₄-Alkyl wie Methyl, Ethyl, n-, i-Propyl, n-, i-, sek.- oder tert.-Butyl. C₃-C₂₄-Cycloalkyl steht beispielsweise für Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cyclotridecyl oder Cyclotetradecyl, vorzugsweise für C₅-C₈-Cycloalkyl wie Cyclopentyl, Cyclohexyl, Cycloheptyl oder Cyclooctyl.

Besonders bevorzugte Verbindungen der Formeln (I) und (II) sind solche, in denen R₁ und/oder R₂ einen sekundären Alkylrest bedeuten wie 1-(C₁-C₉Alkyl)-C₂-C₁₀Alkyl, insbesondere solche, in denen der Rest R₁ eine sogenannte "Schwalbenschwanz-Struktur" aufweist wie 1-Methyl-ethyl, 1-Ethyl-n-propyl, 1-n-Propyl-n-butyl, 1-n-Butyl-n-pentyl, 1-n-Hexyl-1-heptyl, 1-n-Heptyl-1-n-octyl, 1-n-Octyl-1-n-nonyl, 1-n-Nonyl-1-decyl, oder einen aromatischen Rest, insbesondere Phenylrest, ganz besonders bevorzugt C₁-C₆alkylsubstituiertes Phenyl wie 2,6-Di-tert.-butylphenyl und 2,5-Di-tert.-butylphenyl.

C₆-C₁₀Aryl bedeutet beispielsweise Phenyl oder 1- oder 2-Naphthyl, insbesondere Phenyl.

-CO-C₁-C₄Alkyl steht für -CO-Methyl, -CO-Ethyl, -CO-n-Propyl, -CO-i-Propyl, -CO-n-, -i-, -sek.- oder -tert.-Butyl.

C₁-C₄Alkylcarbonsäure-(C₁-C₄Alkyl)-Ester steht beispielsweise für Methylcarbonsäuremethylester, Methylcarbonsäureethylester, Methylcarbonsäure-n-propylester, Methylcarbonsäure-i-propylester, Methylcarbonsäure-n-butylester oder Methylcarbonsäure-i-butylester , Methylcarbonsäure-sek.-butylester oder Methylcarbonsäure-tert.-butylester sowie Ethylcarbonsäuremethylester, Ethylcarbonsäureethylester, Ethylcarbonsäure-n-propylester , Ethylcarbonsäure-i-propylester, Ethylcarbonsäure-n-butylester Ethylcarbonsäure-sek.-butylester oder Ethylcarbonsäure-tert.-butylester, n-Propylcarbonsäuremethylester oder n-Butylcarbonsäuremethylester.

Hal steht beispielsweise für Halogenid und bedeutet Fluorid, Chlorid, Bromid oder lodid.

Besonders bevorzugte Perylen-3,4:9,10-tetracarbonsäurebisimide der allgemeinen Formeln (I) und (II) sind solche, in denen R¹ und R² unabhängig voneinander für 1-n-Hexyl-1-heptyl, 2,5-Di-tert.-butylphenyl, 1-Nonyl-1-decyl- oder 1-n-Butyl-n-pentyl stehen, ganz besonders bevorzugt steht R² für R¹.

Bevorzugte Perylene der vorliegenden Erfindung sind die untenstehenden Verbindungen der Formel 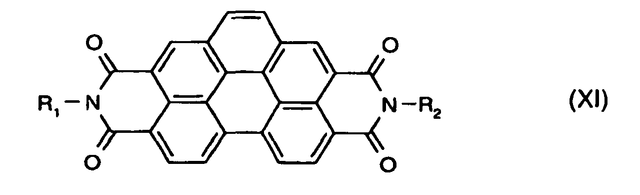 worin
R¹ und R² ganz besonders bevorzugt für 1-n-Hexyl-1-heptyl stehen.

Des weiteren wurde gefunden, dass sich die Verbindung (VIII) der Formel 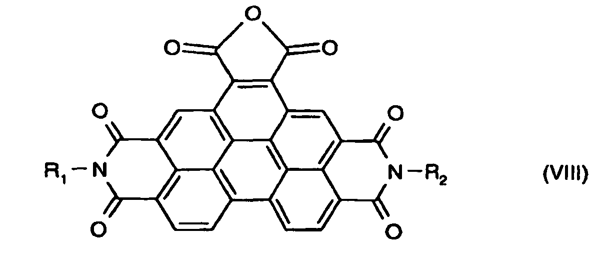 worin
R¹ und R² die unter der Formel (I) angegebene Bedeutung haben und bevorzugt unabhängig voneinander für 1-Hexyl-1-heptyl, 2,5-Di-tert.-butylphenyl, 1-Nonyl-1-decyl- oder 1-Butyl-pentyl stehen
in ein Dicarbonsäurederivat (XXVIIa) 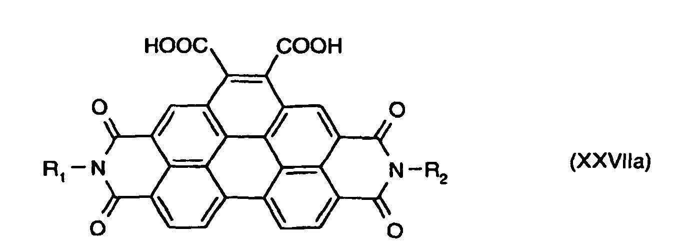 und durch Veresterung in den Dicarbonsäureester der Formel (XXVIIb) 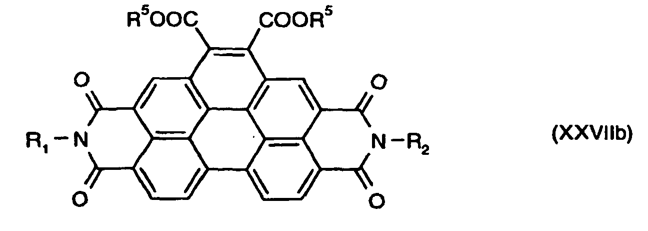 worin
R⁵ für C₁-C₂₄Alkyl oder C₃-C₂₄Cycloalkyl steht, überführen lässt. ,

Eine weitere Ausführungsform der vorliegenden Erfindung betrifft daher auch ein Verfahren zur Herstellung von Verbindungen der Formel (XXVIIa) durch Hydrolyse eines Anhydrids, indem man als Anhydrid die Verbindung (VIII) einsetzt und diese mit einer Säure oder einer Base, bevorzugt in wässrigem Milieu, zur Reaktion bringt.

Die Hydrolyse von Anhydriden ist bekannt und beispielsweise in Survey of Organic Synthesis, von Calvin A. Buehler und Donald E. Pearson, Wiley-Intersience, USA, (1970) beschrieben.

Vorzugsweise nimmt man die Umsetzung bei Reaktionstemperaturen im Bereich von -10 bis 200°C, besonders bevorzugt von 0 bis 150°C vor. Der Erfolg der Umsetzung ist nach bisherigen Beobachtungen nicht von der Wahl des Druckbereiches abhängig. Der Einfachheit halber führt man üblicherweise die Umsetzung bei Atmosphärendruck durch, jedoch können auch Drücke im Bereich von 10 kPa bis 10 MPa gewählt werden. Die Reaktionszeiten wählt man, je nach gewählter Reaktionstemperatur, bevorzugt im Bereich von 1 h bis 24 h.

Als Säure kann man beispielsweise eine anorganische Säure wie Salzsäure, Schwefelsäure, Lewis-Säuren wie Bortrifluorid oder organische Säuren wie Methansulfonsäuren, Ameisensäure oder para-Toluolsulfonsäure einsetzen.

Als Basen kann man beispielsweise Alkalimetallalkoholate wie Natrium- oder Kalium-methylat oder Natrium- oder Kaliumethylat sowie Alkalimetallcarbonate und Alkalimetallhydrogencarbonate wie Natriumcarbonat oder Kaliumcarbonat wie Natriumhydrogen-carbonat oder Kaliumhydrogencarbonat sowie Alkalimetallhydroxide wie Natriumhydroxid, Kaliumhydroxid, insbesondere Kaliumhydroxidpulver (85 Gew.-% Kaliumhydroxid und 15 Gew.-% Wasser), des weiteren Lithiumaluminiumhydrid, Kalium-tert.-butylat, Triethylamin, Aluminiumalkoxid sowie nicht-nucleophile Basen wie 1,8-Diazabicycl[5.4.0]undec-7-en (DBU), 1,5-Diazabicycl[4.3.0]non-5-en ((DBN) oder N,N,N',N'-Tetramethylethylendiamin (TMEDA) verwenden.

Die Säure oder Base setzt man in der Regel in einem Molverhältnis der Säure oder Base zur Verbindung (VIII) im Bereich von 0,1:1 bis 20:1 ein.

Es hat sich als vorteilhaft erwiesen, die Umsetzung in Gegenwart eines organischen Lösungsmittels durchzuführen. Beispielsweise kommen als Lösungsmittel die oben genannten in Betracht.

Das Gewichtsverhältnis von Summe der Reaktanden zum Lösungsmittel liegt üblicherweise im Bereich von 0,001 bis 20 Gew.-%, bevorzugt von 0,001 bis 10 Gew.-%.

In einer weiteren bevorzugten Ausführungsform nimmt man die Umsetzung in einer Schutzgasatmosphäre vor. Bevorzugte Schutzgase sind beispielsweise Stickstoff und die Edelgase wie Helium oder Argon.

Ein weiterer Erfindungsgegenstand der vorliegenden Erfindung betrifft ein Verfahren zur Herstellung von Diester-Derivaten (XXVIIb) durch Veresterung einer Dicarbonsäure, indem man Verbindungen der Formel (XXVIIa), mit einem Alkohol der Formel (XXVIII), HO-R⁵, oder einem Alkylhalogenid der Formel (XXIX), Hal-R⁵, in Gegenwart einer Säure oder einer Base zur Reaktion bringt.

Als Basen und Säuren sind die oben aufgeführten geeignet.

Methoden zur Veresterung von Carbonsäuren sind bekannt, und beispielsweise in Survey of Organic Synthesis, (1970) beschrieben.

Eine besondere Ausgestaltung des erfindungsgemässen Verfahrens betrifft die Umsetzung mit einer Base. Insbesondere nicht-nucleophile Basen haben sich als vorteilhaft erwiesen.

In der Regel liegt das Molverhältnis von Base zur Verbindung (XVIIa) im Bereich von 100:1 bis 1:100, bevorzugt im Bereich von 20:1 bis 1:20.

Das Molverhältnis des Alkylhalogenids zur Base liegt in der Regel im Bereich von 5:1 bis 1:5, bevorzugt im Bereich von 2:1 bis 1:2.

Es hat sich als vorteilhaft erwiesen, die erfindungsgemässe Reaktion in Gegenwart von Lösungsmitteln durchzuführen. Geeignete Lösungsmittel sind die oben aufgeführten.

In der Regel wird das Lösungsmittel in einer solchen Menge eingesetzt, die ausreicht, um die Verbindung (XVIIa) lösen.

Ferner wurde gefunden, dass sich die Dicarbonsäurederivate (XVIIa) und Anhydride der Formel (VIII) decarboxylieren lassen.

Ein weiterer Erfindungsgegenstand der vorliegenden Erfindung betrifft daher auch ein Verfahren zur Herstellung der Perylenbisimide der Formel (XI), durch Decarboxylierung einer Dicarbonsäure oder eines Anhydrids mit Kupfer oder einer kupferhaltigen Verbindung, indem man die Verbindung der Formeln (XXVIIa) oder (VIII) in Gegenwart eines Lösungsmittels zur Reaktion bringt.

Die Decarboxylierung von Carbonsäuren kann bekanntermassen mit Kupfer in Gegenwart von Chinolin durchgeführt werden (siehe Survey of Organic Synthesis, (1970), 144, 145).

Kupfer steht beispielsweise für Kupferpulver und als kupferhaltige Verbindungen kann man Cu(I)- oder Cu(II)-Salze, bevorzugt Kupfer(I)- oder -(II)-oxid, Kupferchromit oder Kupfersulfat, einsetzen, bevorzugt verwendet man Kupferpulver.

Kupfer oder eine kupferhaltige Verbindung setzt man in der Regel im Überschuß ein. Bevorzugt liegt das Molverhältnis des Kupfers (ebenso Kupfer-Äquivalent in kupferhaltigen Verbindungen) zur Verbindung der Formel (XVIIa) oder (VIII) im Bereich von 1,1:1 bis 20:1, besonders bevorzugt im Bereich von 1,3:1 bis 15:1.

Vorzugsweise nimmt man die Umsetzung bei Reaktionstemperaturen im Bereich von 80 bis 200°C, besonders bevorzugt von 120 bis 180°C vor. Der Erfolg der Umsetzung ist nach bisherigen Beobachtungen nicht von der Wahl des Druckbereiches abhängig. Der Einfachheit halber führt man üblicherweise die Umsetzung bei Atmosphärendruck durch, jedoch können auch Drücke im Bereich von 10 kPa bis 10 MPa gewählt werden. Die Reaktionszeiten wählt man, je nach gewählter Reaktionstemperatur, bevorzugt im Bereich von 1 h bis 48 h, besonders bevorzugt im Bereich von einem 1 h bis 10 h.

Es hat sich als vorteilhaft erwiesen, die Umsetzung in Gegenwart eines organischen Lösungsmittels durchzuführen. Beispielsweise kommen als Lösungsmittel die weiter oben bei dem Verfahren zur Herstellung von Verbindung der Formel (VIII) genannten in Frage. Besonders bevorzugte Lösungsmittel sind stickstoffhaltige Lösungsmittel, und insbesondere bevorzugt sind Chinolin und 3-Picolin.

Das Gewichtsverhältnis von Summe der Reaktanden zum Lösungsmittel liegt üblicherweise im Bereich von 0,001 bis 20 Gew.-%, bevorzugt von 0,001 bis 10 Gew.-%.

In einer weiteren bevorzugten Ausführungsform nimmt man die Umsetzung in einer Schutzgasatmosphäre vor. Bevorzugte Schutzgase sind beispielsweise Stickstoff und die Edelgase wie Helium oder Argon.

Perylen-monoanhydrid-monoimide können als Ausgangsverbindungen zur Herstellung von Perylenbisimiden in enger Analogie zu bekannten Methoden verwendet werden. Beispielsweise ist in Heterocycles (1995), 40, 477-500 beschrieben, dass man Anhydrid-Derivate der Perylene mit primären Aminen zu den entsprechenden Imiden umsetzen kann.

Des weiteren kann man die erfindungsgemässen Anhydrid-Derivate als Fluoreszenzmarker einsetzen.

Bisher sind die Nitroderivate von Derivaten der Perylentetracarbonsäure, insbesondere monosubstiuierte, nur in ungenügenden Ausbeuten zugänglich (siehe beispielsweise J. Org. Chem. USSR (Engl. Translation) 1980, 16, 762-766).

Eine weitere Ausführungsform der vorliegenden Erfindung betrifft die Verwendung der erfindungsgemässen Perylene (I) und (II) als Farbmittel, insbesondere als Pigmente und Farbstoffe, nach in der Regel jeweils an sich bekannten Methoden, bevorzugt
(a) zur Masse-Färbung von Polymeren, wobei man als Polymere Polyvinylchlorid, Celluloseacetat, Polycarbonat, Polyamid, Polyurethan, Polyimid, Polybenzimidazol, Melaminharz, Silikon, Polyester, Polyether, Polystyrol, Polymethylmethacrylat, Polyethylen, Polypropylen, Polyvinylacetat, Polyacrylnitril, Polybutadien, Polychlorbutadien oder Polyisopren bzw. die Copolymeren der genannten Monomeren einsetzen kann;
(b) als Küpenfarbstoffe oder Beizenfarbstoffe, z.B. zur Färbung von Naturstoffen sowie insbesondere von Papier, Holz, Stroh, Leder, Fellen oder natürlichen Fasermaterialien wie Baumwolle, Wolle, Seide, Jute, Sisal, Hanf, Flachs oder Tierhaare (z.B. Roßhaar) und deren Umwandlungsprodukte wie die Viskosefaser, Nitratseide oder Küpferrayon, wobei bevorzugte Salze zum Beizen Aluminium-, Chrom- und Eisensalze sind;
(c) bei der Herstellung von Farben, Lacken, insbesondere Automobillacken, Anstrichstoffen, Papierfarben, Druckfarben, Tinten, insbesondere zum Einsatz in Tintenstrahldruckern, bevorzugt in homogener Lösung als fluoreszierende Tinte, und für Mal- und Schreib-Zwecke, sowie in der Elektrophotographie z.B. für Trockenkopiersysteme (Xerox-Verfahren) und Laserdrucker;
(d) für Sicherheitsmarkierungs-Zwecke wie für Schecks, Scheckkarten, Geldscheine, Coupons, Dokumente, Ausweispapiere und dergleichen, bei denen ein besonderer, unverkennbarer Farbeindruck erzielt werden soll;
(e) als Zusatz zu Farbmitteln wie Pigmenten und Farbstoffen, bei denen eine bestimmte Farbnuance erzielt werden soll, bevorzugt sind besonders leuchtende Farbtöne;
(f) zum Markieren von Gegenständen zum maschinellen Erkennen dieser Gegenstände über die Fluoreszenz, bevorzugt ist die maschinelle Erkennung von Gegenständen zum Sortieren, z.B. auch für das Recycling von Kunststoffen, wobei alphanumerische Aufdrucke oder Barcodes vorzugsweise eingesetzt werden;
(g) zur Frequenzumsetzung von Licht, z.B. um aus kurzwelligem Licht längerwelliges, sichtbares Licht zu machen oder zur Frequenzverdopplung und Frequenzverdreifachung von Laserlicht in der nichtlinearen Optik;
(h) zur Herstellung von passiven Anzeigeelementen für vielerlei Anzeige-, Hinweis- und Markierungszwecke, z.B. passive Anzeigeelemente, Hinweis- und Verkehrszeichen, wie Ampeln;
(i) als Ausgangsmaterial für supraleitende organische Materialien (über π-π-Wechselwirkungen, nach Dotierung mit z.B. lod erhält man üblicherweise eine intermediäre Ladungsdelokalisierung);
(j) zur Feststoff-Fluoreszenz-Markierung;
(k) für dekorative und künstlerische Zwecke;
(l) zu Tracer-Zwecken, z.B. in der Biochemie, Medizin, Technik und Naturwissenschaft, wobei die erfindungsgemässen Farbmittel kovalent mit Substraten oder über Nebenvalenzen wie Wasserstoffbrückenbindungen oder hydrophobe Wechselwirkungen (Adsorption) verknüpft sein können; Beispiele sind Protein-Farbstoff-Kombinationen, Antikörper-Farbstoff-Kombinationen oder DNA- oder RNA-Farbstoff-Kombinationen,
(m) als Fluoreszenzfarbstoffe in hochempfindlichen Nachweisverfahren (siehe C. Aubert, J. Fünfschilling, 1. Zschokke-Gränacher und H. Langhals, Z. Analyt. Chem. 1985, 320, 361), insbesondere als Fluoreszenzfarbstoffe in Szintillatoren;
(n) als Farbstoffe oder Fluoreszenzfarbstoffe in optischen Lichtsammelsystemen, in Fluoreszenzquantenzählern, in Fluoreszenz-Solarkollektoren (siehe H. Langhals, Nachr. Chem. Tech. Lab. 1980, 28, 716), in Fluoreszenz-aktivierten Displays (siehe W. Greubel und G. Baur, Elektronik 1977, 26, 6), in Kaltlichtquellen zur lichtinduzierten Polymerisation zur Darstellung von Kunststoffen, zur Materialprüfung, z.B. bei der Herstellung von Halbleiterschaltungen, zur Untersuchung von Mikrostrukturen von integrierten Halbleiterbauteilen, in Photoleitern, in fotografischen Verfahren, in Anzeige-, Beleuchtungs- oder Bildwandlersystemen, bei denen die Anregung durch Elektronen, Ionen oder UV-Strahlung erfolgt, z.B. in Fluoreszenzanzeigen, Braunschen Röhren oder in Leuchtstoffröhren, als Teil einer integrierten Halbleiterschaltung, die Farbstoffe als solche oder in Verbindung mit anderen Halbleitern z.B. in Form einer Epitaxie, enthalten, in Chemilumineszenzsystemen, z.B. in Chemilumineszenz-Leuchtstäben, in Lumineszenzimmunassays oder anderen Lumineszenznachweisverfahren, als Signalfarben, bevorzugt zum optischen Hervorheben von Schriftzügen und Zeichnungen oder anderen graphischen Produkten, zum Kennzeichnen von Schildern und anderen Gegenständen, bei denen ein besonderer optischer Farbeindruck erreicht werden soll, in Farbstoff-Lasern, bevorzugt als Fluoreszenzfarbstoffe zur Erzeugung von Laserstrahlen sowie als Q-Switch-Schalter;
(o) als Rheologieverbesserer sowie
(p) zur Modifizierung anorganischer Feststoffe wie Aluminiumoxid, Siliciumoxid wie zum Beispiel in Zeolithkäfigen, Titandioxid, Zinnoxid, Magnesiumoxid ("Steinholz"), Silikate, Tonmineralien, Kalk-, Gips- oder Zement-haltige Oberflächen wie Anstriche oder Putzoberflächen, bei denen die freie Carboxylfunktion eine besondere Haftung auf der Oberfläche gewährleistet, oder
(q) als NIR-Farbstoffe für Informationstechnologien.

Insbesondere die Verbindungen, worin R₁ und/oder R₂ für 1-Butyl-pentyl, 1-Hexyl-1-heptyl, 1-Heptyl-1-octyl, 1-Octyl-1-nonyl, 1-Nonyl-1-decyl und 2,5-Di-tert.-butylphenyl stehen, zeichnen sich durch ihre gute Löslichkeit aus.

### Beispiele

Beispiel 1a: 1-Nitro-N,N'-bis(1-butylpentyl)-perylen-3,4:9,10-bis(dicarboximid) (XL):
   N,N'-Bis(1-butylpentyl)-perylen-3,4:9,10-bis(dicarboximid) (hergestellt wie in Chem. Ber. 1988, 121, 225-230 beschrieben) 2,00 g (3,12 mmol), 30 ml Dichlormethan, eine Lösung aus N₂O₄ in Dichlormethan, 37 ml (0,085 molar, 3,15 mmol, hergestellt wie weiter unten beschrieben) und Methansulfonsäure, 0,02 ml (0,30 mmol), werden bei 25°C 6 h lang umgesetzt. Das Reaktionsgemisch wird auf ca. 2 ml eingeengt. Der Rückstand wird in 10 ml Toluol aufgenommen und chromatographisch an Kieselgel/Toluol aufgearbeitet.
   Ausb. 2,1 g (98%) (XL) als dunkelrotes Pulver, Schmp. 304 °C, R_{f} (Kieselgel/Toluol): 0,31.
Beispiel 1b: 1-Nitro-N,N'-bis(1-hexylheptyl)-perylen-3,4:9,10-bis(dicarboximid) (XLI):
   N,N'-Bis-(1-Hexylheptyl)-perylen-3,4:9,10-bis(dicarboximid) (hergestellt wie in Chem. Ber. 1988, 121, 225-230 beschrieben), 2,07 g (2,75 mmol), eine Lösung aus N₂O₄ in Dichlormethan, 14,0 ml (0,198 molare, 2,77 mmol N₂O₄/ hergestellt, wie unten beschrieben), und Methansulfonsäure, 0,05 ml (0,77 mmol), werden bei 25°C 7 h lang umgesetzt. Das Reaktionsgemisch wird auf ca. 2 ml eingeengt. Der Rückstand wird in 10 ml Toluol aufgenommen und chromatographisch an Kieseigel/Toluol aufgearbeitet.
   Ausb. 2,04 g (93%) (XLI) dunkelrotes Pulver, Schmp. 121°C, R_{f} (Kieselgel/Trichlormethan): 0,84. - R_{f} (Kieselgel/Toluol): 0,63.
Beispiel 1c: 1-Nitro-N,N'-bis(2,5-di-tert-butylphenyl)-perylen-3,4:9,10-bis(dicarboximid)(XLII);
   N,N'-Bis(2,5-di-*tert*-butylphenyl)-perylen-3,4:9,10-bis(dicarboximid) (hergestellt wie in Chem. Ber. 1985, 118, 4641-4645 beschrieben), 800 mg (1,04 mmol), Dichlormethan, 30 ml, eine Lösung aus N₂O₄ in Dichlormethan, 21 ml (0,05-molar,1,05 mmol, hergestellt wie unten beschrieben), und Methansulfonsäure, 0,05 ml (0,77 mmol), werden bei 25 °C 4 h lang umgesetzt. Das Rohprodukt wird auf ca. 1 ml eingeengt. Der Rückstand wird in 10 ml Trichlormethan aufgenommen und chromatographisch an Kieselgel/Trichlormethan aufgearbeitet. Ausb.: 800 mg (94%)(XLII) dunkelrotes Kristallpulver, Schmp. > 300 °C,
   R_{f} (Kieselgel/Trichlormethan): 0,45.
Beispiel 1d: 1-Nitro-N-(1-hexylheptyl)-perylen-3,4:9,10-tetracarbonsäure-3,4-anhydrid-9,10-imid (XLIII) und 12-Nitro-N-(1-hexylheptyl)-perylen-3,4:9,10-tetracarbonsäure-3,4-anhydrid 9,10-imid (XLIV): N-(1-Hexylheptyl)-perylen-3,4:9,10-tetracarbonsäure-3,4-anhydrid-9,10-imid, 100 mg (0,175 mmol) (hergestellt wie in Chem. Ber. 124, 529-535 beschrieben), Dichlormethan, 50 ml, eine Lösung aus N₂O₄ in Dichlormethan 5,5 ml (0,03-molaren, 0,165 mmol, hergestellt wie unten beschrieben), und Methansulfonsäure, 0,05 ml (0,77 mmol), werden bei 25°C 4 h lang umgesetzt. Das Rohprodukt wird auf ca. 1 ml eingeengt. Der Rückstand wird in 20 ml Trichlormethan aufgenommen und chromatographisch an Kieselgel/Trichlormethan/7% Eisessig aufgearbeitet.
   Ausb.: 83 mg (77%) einer Mischung aus (XLIII) und (XLIV) als dunkelrotes Pulver, Schmp.: 189-191°C, R_{f} (Kieselgel/Dichlormethan /2% Eisessig): 0,73.
   Herstellung einer N₂O₄-Lösung: Pb(NO₃)₂ wird einige Minuten auf 250 bis 600°C erhitzt und das entweichende N₂O₄-Gas in Dichlormethan bis zur Sättigung eingeleitet. Zur Konzentrationsbestimmung der Lösung aus N₂O₄ in Dichlormethan werden 10 ml dieser Lösung mit 10 ml 30 Gew.-%iger Wasserstoffperoxid geschüttelt, bis die hellbraune Farbe verschwindet. Man erhält ein Zweiphasengemisch. Die untere Dichlormethanphase wird abgetrennt. Die obere wässerige Phase wird mit Phenolphthalein als Indikator gegen NaOH (0,1 N) zur Bestimmung des Salpetersäuregehalts titriert.
Beispiel 2a: N,N'-Bis(1-hexylheptyl)-perylen-3,4:9,10-bis(dicarboximid)-1-diethylphosphonat (XLV) und N,N'-Bis(1-hexylheptyl)-pyrrolo[2,3,4,5-hik]perylen-3,4:8,9-bis(dicarboximid) (XLVI):
   (XLI), hergestellt analog Beispiel 1b, 150 mg (0,188 mmol) und Triethylphosphit, 5 ml (29,1 mmol), werden unter Argonatmosphäre bei 130 °C 5 h lang umgesetzt. Die Reaktionsmischung wird anschliessend mit 1N Salzsäure, 150 ml, bei ca. 25°C ca. 12 h umgesetzt. Es entsteht ein dunkelroter Niederschlag. Der Niederschlag wird abgesaugt und getrocknet. Zur weiteren Reinigung wird der Niederschlag in Trichlormethan aufgenommen und und chromatographisch an Aluminiumoxid/Trichlormethan aufgearbeitet:
   1.Fraktion: Ausb. 43 mg (26%) (XLV), Schmp. 61-63°C, R_{f} (Al₂O₃/Trichlormethan):,0.82;
   2. Fraktion: Ausb. 91 mg (63%) (XLVI), Schmp. 204 - 205 °C, R_{f} (Al₂O₃/Trichlormethan): 0,42.
Beispiel 2b: N,N'-Bis(1-butylpentyl)-perylen-3,4:9,10-bis(dicarboximid)-1-diethylphosphonat (XLVII) und N,N'-Bis(1-butylpentyl)-pyrrolo[2,3,4,5-hik]perylen-3,4;8,9-bis(dicarboximid) (XLVIII):
   (XL), hergestellt analog zu Beispiel 1a, 300 mg (0,437 mmol) und Triethylphosphit, 10 ml (9,69 g, 58,3 mmol), werden unter Argonatmosphäre bei 130°C 3 h lang umgesetzt. Die Reaktionsmischung wird anschliessend mit 1 N Salzsäure, 100 ml, versehen und bei ca. 25°C ca. 12 h umgesetzt. Es entsteht ein dunkelroter Niederschlag. Der Niederschlag wird abgesaugt und getrocknet. Zur weiteren Reinigung wird der Niederschlag in Trichlormethan aufgenommen und chromatographisch an Aluminiumoxid (der Aktivitätsstufe II)/Trichlormethan/1% Ethanol aufgearbeitet:
   1. Fraktion: Ausb. 109 mg (32%) (XLVII), Schmp. 122 - 124 °C, R_{f} (Al₂O₃/Trichlormethan): 0,62.
   Fraktion: Ausb. 160 mg (56%) (XLVIII), Schmp. > 300 °C, R_{f} (Al₂O₃ neutral/Trichlormethan): 0,24.
Beispiel 3: N,N"-Bis(1-butylpentyl)-N'-methyl-pyrrolo[2,3,4,5-hik]perylen-3,4;8,9-bis(dicarboximid) (XLIX) Methode A:
   (XLVIII), hergestellt analog zu Beispiel 2b, 100 mg (0,153 mmol), Ethanol, 10 ml, und Kaliumhydroxidpulver, 17 mg (0,258 mmol), werden mit lodmethan, 0,5 ml (8 mmol) bei ca. 25°C ca. 12 h umgesetzt. Anschliessend wird das Ethanol abdestilliert. Der Rückstand des Reaktionsgemischs wird in Trichlormethan aufgenommen und mehrmals mit Wasser gewaschen. Die organische Lösungsmittelphase wird anschliessend mit Magnesiumsulfat getrocknet und dann zur Trockene eingeengt. Der Rückstand wird in Trichlormethan aufgenommen und chromatographisch an Kieselgel/Trichlormethan aufgearbeitet:
   Ausb. 93 mg (91%) (XLIX) kirschrotes Pulver.
   Methode B: (XLVIII), 100 mg (0,153 mmol) und Ethanol, 10 ml, werden mit Kaliumhydroxidpulver, 17,0 mg (0,258 mmol), umgesetzt. Die entstandene violette Lösung wird eingeengt, und der Rückstand in N-Methylpyrrolidon aufgenommen und mit lodmethan, 0,1 ml (1,6 mmol)) bei 25°C 12 h umgesetzt. Anschliessend giesst man das Reaktionsgemisch in Wasser, fügt dann soviel Ethanol hinzu, bis sich das N-Methylpyrrolidon aufgelöst hat, und rührt bei 25°C 12 h lang. Anschliessend wird Ethanol abdestilliert. Der Rückstand des Reaktionsgemischs wird in Trichlormethan aufgenommen und mehrmals mit Wasser gewaschen. Die organische Lösungsmittelphase wird anschliessend mit Magnesiumsulfat getrocknet und dann zur Trockene eingeengt. Der Rückstand wird in Trichlormethan aufgenommen und chromatographisch an Kieselgel/Trichlormethan aufgearbeitet. Ausb. 93 mg (91%) als kirschrotes Pulver, (XLIX), Schmp. > 300 °C,
   R_{f} (Kieselgel/Trichlormethan): 0,61. - R_{f} (Al₂O₃/Trichlormethan): 0,77.
Beispiel 4: N,N"-Bis(1-hexylheptyl)-N'-benzyl-pyrrolo[2,3,4,5-hik]perylen-3,4:8,9-bis(dicarboximid) (L), Methode A:
   (XLVI), hergestellt analog zu Beispiel 2a, 100 mg (0,13 mmol), Ethanol, 10 ml, und Kaliumhydroxidpulver, 11 mg (0,167 mmol), werden mit Benzylbromid, 222 mg (1,3 mmol), bei 25°C 12 h lang umgesetzt. Anschliessend wird Ethanol abdestilliert. Der Rückstand des Reaktionsgemischs wird in Trichlormethan aufgenommen und chromatographisch an Kieselgel/(Petrolether:Trichlormethan) (5:1) und dann an Kieselgel/Trichlormethan aufgearbeitet. Ausb. 83.0 mg (74%) (L) als hellrotes Pulver.
   Methode B: N,N"-Bis(1-hexylheptyl)-pyrrolo[2,3,4,5-hik]perylen-3,4;8,9-bis(dicarboximid) (XLVI), hergestellt analog zu Beispiel 2a, 100 mg (0,130 mmol), Tetrahydrofuran, 10 ml und DBU ,30 mg (0,197 mmol) werden mit Benzylbromid 222 mg (1,30 mmol)m bei ca. 25°C umgesetzt, bis das Ausgangsmaterial dünnschichtchromatographisch nicht mehr nachweisbar ist. Anschliessend wird Tetrahydrofuran abdestilliert und analog zu Methode A aufgearbeitet. Ausb. 97 mg (87%) (L), Schmp. 134-135°C, R_{f} (Kieselgel/Trichlormethan): 0,62.
Beispiel 5: N,N"-Bis(1-hexylheptyl)-N'-carbethoxymethyl-pyrrolo[2,3,4,5-hik]perylen-3,4:8,9-bis(dicarboximid) (LI):
   (XLVI), hergestellt analog zu Beispiel 2a, 105 mg (0,137 mmol), Tetrahydrofuran, 15 ml und DBU, 31 mg, (0,204 mmol), werden mit Bromessigsäureethylester, 46 mg (0,277 mmol), bei ca. 25°C umgesetzt, bis das Ausgangsmaterial dünnschichtchromatographisch nicht mehr nachweisbar ist. Anschliessend wird der Ansatz mit 2N Salzsäure und dann mit so viel Aceton versetzt, bis der überschüssige Bromessigsäureethylester in Lösung geht. Der dabei entstandene rote Niederschlag wird abfiltriert, der Filterrückstand getrocknet und chromatographisch an Kieselgel/Trichlormethan aufgearbeitet.
   Ausb. 95 mg (81%) (LI) als leuchtend rotes Pulver, Schmp. 110-111°C,
   R_{f} (Kieseigel/Trichlormethan): 0,56.
Beispiel 6a: N,N"-Bis(1-hexylheptyl)-N'-acetyl-pyrrolo[2,3,4,5-hik]perylen-3,4:8,9-bis(dicarboximid) (LII):
   (XLVI), hergestellt analog zu Beispiel 2a, 56 mg (0,07 mmol), gelöst in Tetrahydrofuran und DBU, 17 mg (0,11 mmol), werden mit Acetylchlorid, 12 mg (0,15 mmol), bei 25°C umgesetzt, bis das Ausgangsmaterial dünnschichtchromatographisch nicht mehr nachweisbar ist. Anschliessend wird das Reaktionsgemisch mit Wasser, 50 ml, bei 25°C 12 h lang umgesetzt. Der entstandene Niederschlag wird abfiltriert, der Filterrückstand isoliert und getrocknet und anschliessend chromatographisch an Kieselgel/Trichlormethan aufgearbeitet. Ausb. 43 mg (73%) (LII) als leuchtend rotes Pulver, Schmp. > 300 °C,
   R_{f} (Kieselgel/Trichlormethan): 0,62.
Beispiel 6b: N,N"-Bis(1-butylpentyl)-N'-acetyl-pyrrolo[2,3,4,5-hik]perylen-3,4;8,9-bis(dicarboximid) (LIII)
   (XLVIII), hergestellt analog zu Beispiel 2b, 48 mg (0,073 mmol), gelöst in Tetrahydrofuran und DBU, 22 mg (0,145 mmol), werden mit Acetylchlorid, 10 mg (0,128 mmol), bei 25°C umgesetzt, bis das Ausgangsmaterial dünnschichtchromatographisch nicht mehr nachweisbar ist. Anschliessend wird das Reaktionsgemisch mit Wasser, 50 ml, bei 25°C 12 h lang umgesetzt. Der entstandene Niederschlag wird abfiltriert, der Filterrückstand isoliert und getrocknet und anschliessend chromatographisch an Kieselgel/Dichlormethan aufgearbeitet. Ausb. 35 mg (69%) (LIII) als leuchtend rotes Pulver, Schmp. > 300°C,
   R_{f} (Kieselgel/Trichlormethan): 0,23.
Beispiel 7:N,N"-Bis(1-hexylheatyl)-N'-benzoyl-pyrrolo[2,3,4,5-hik]perylen-3,4;8,9-bis(dicarboximid) (LIV):
   (XLVI), hergestellt analog zu Beispiel 2a, 80,0 mg (0.104 mmol), gelöst in Tetrahydrofuran und DBU, 25 mg (0,164 mmol), werden mit Benzoylchlorid, 44 mg (0,314 mmol), bei ca. 25°C umgesetzt, bis das Ausgangsmaterial dünnschichtchromatographisch nicht mehr nachweisbar ist. Anschliessend wird das Reaktionsgemisch mit Wasser, 50 ml, bei ca. 25°C ca. 12 h lang umgesetzt. Der entstandene Niederschlag wird abfiltriert. Der Filterrückstand wird isoliert und getrocknet und anschliessend chromatographisch an Kieselgel/Trichlormethan aufgearbeitet. Ausb. 84 mg (92%) (LIV) als leuchtend rotes Pulver, Schmp. 181 - 183 °C, R_{f} (Kieselgel/Trichlormethan): 0,51.
Beispiel 8:1-Brom-N,N'-bis(1-hexylheptyl)-perylen-3,4:9,10-bis(dicarboximid) (LV):
   N,N'-Bis(1-hexylheptyl)-perylen-3,4:9,10-bis(dicarboximid), hergestellt analog zu Chem. Ber. 1988, 121, 225-230 beschrieben), 200 mg (0,265 mmol), gelöst in Chlorbenzol, Brom, 1 ml (39,3 mmol), und wasserfreies Kaliumcarbonat, 520 mg (3,77 mmol), werden bei 60°C 24 h lang umgesetzt. Anschliessend wird Chlorbenzol abdestilliert, und der Destillationsrückstand chromatographisch an Kieselgel/Trichlormethan aufgearbeitet. Ausb. 190 mg (86%) (LV) als dunkelrotes Pulver, Schmp. 136-138°C. R_{f} (Kieselgel/Trichlormethan): 0,79.
Beispiel 9: 1-Amino-N,N'-bis(1-hexylheptyl)-perylen-3,4:9,10-bis(dicarboximid) (LVI):
   Methode A: (XLI), hergestellt analog zu Beispiel 1b, 100 mg (0,125 mmol), Eisen, 50 mg (0,893 mmol), Ethanol (oder n-Butanol oder Tetrahydrofuran) werden mit konz. Salzsäure, 0,5 ml, unter Rückfluss umgesetzt bis dünnschichtchromatographisch kein (XLI) mehr nachweisbar ist (ca. 10 - 30 Minuten). Anschliessend wird der entstandene Niederschlag mit Wasser ausgefällt und abfiltriert. Der Filterrückstand wird getrocknet und dann chromatographisch an Kieselgel/Trichlormethan aufgearbeitet.
   Ausb. 78,0 mg (81%) (LVI) als dunkelblaues Pulver
   Methode B: (XLI), hergestellt analog Beispiel 1 b, 20 mg (0,025 mmol), gelöst in Tetrahydrofuran, Triethylamin, 0,200 ml (1,44 mmol), und Palladium/Kohle, 5 mg (5%Gew.-%ig), werden bei Siedetemperatur ca. 30 min lang umgesetzt. Anschliessend wird Ameisensäure, 0,040 ml (1,06 mmol) und Palladium/Kohle 5 mg (5 gew.-%ig) zugefügt und bei Siedetemperatur 25 min lang umgesetzt, bis dünnschichtchromatographisch kein (XLI) mehr nachweisbar ist. Dann wird analog zur Methode A aufgearbeitet. Ausb. 14 mg (73%) (LVI) als dunkelblaues Pulver, Schmp. 93 - 95 °C, R_{f} (Kieselgel/Trichlormethan): 0,34.
Beispiel 10:1-Dimethylamino-N,N'-bis(1-hexylheptyl)-perylen-3,4:9,10-bis(dicarboximid) (LVII), Methode A: (LVI), hergestellt analog zu Beispiel 9, 34 mg (0,044 mmol), gelöst in Ameisensäure, 5 ml, und Dimethylformamid, 2 ml, werden mit Formalinlösung, 0,5 ml (35 gew.-%ig), bei 85°C 24 h lang umgesetzt. Anschliessend wird das Reaktionsgemisch mit Wasser versetzt und dann mit Natriumcarbonat schwach basisch gestellt. Die Reaktionsmischung wird filtriert und der Filterrückstand isoliert, getrocknet und anschliessend chromatographisch an Kieselgel/Trichlormethan aufgearbeitet. Ausb.: 21 mg (60%) (LVII)
   Methode B: (LVI), hergestellt analog zu Beispiel 9, 50 mg (0,065 mmol), gelöst in Toluol, Kaliumhydroxidpulver, 15 mg (0,228 mmol) und Triethylbenzylammoniumchlorid, 5 mg (0,022 mmol), werden mit Methyliodid, 0,04 ml (0,642 mmol), bei 25°C 12 h lang umgesetzt. Anschliessend wird das Reaktionsgemisch mit dem Lösungsmittel verdünnt und dreimal mit Wasser gewaschen. Die organische Lösungsmittelphase wird von der Wasserphase getrennt und über Magnesiumsulfat getrocknet und anschliessend das Lösungsmittel destillativ entfernt. Anschliessend wird chromatographisch an Kieselgel/Trichlormethan und dann an Kieselgel/Dichlormethan aufgearbeitet. Nach der Elution des (LVII) erhält man noch das blaugrüne Monomethylierungsprodukt, 1-Methylamino-N,N'-bis(1-hexylheptyl)-perylen-3,4:9,10-bis(dicarboximid), (LVIII), als Nebenprodukt. Ausb. 40 mg (77%) (LVII) als dunkelgrüner Feststoff, Schmp. 72 - 74 °C, R_{f} (Kieselgel/Trichlormethan): 0,63.
Beispiel 11a: 1-Amino-N,N'-bis(1-butylpentyl)-perylen-3,4:9,10-bis(dicarboximid) (LIX):
   (XL), hergestellt analog zu Beispiel 1a, 100 mg (0,146 mmol), gelöst in Tetrahydrofuran, Eisen, 50 mg (0,893 mmol), und konzentrierte Salzsäure, 0,5 ml, werden bei Siedetemperatur umgesetzt, bis dünnschichtchromatographisch kein (XL) mehr nachweisbar ist (nach ca. 15 min). Anschliessend wird das Reaktionsgemisch mit Wasser und 2N Salzsäure umgesetzt. Der entstandene Niederschlag wird filtriert. Der Filterrückstand enthaltend (LIX) isoliert und getrocknet.
Beispiel 11b:1-Dimethylamino-N,N'-bis(1-butylpentyl)-perylen-3,4:9,10-bis(dicarboximid) (LX)
   (LIX), hergestellt nach Beispiel 11a, wird in Dichlormethan gelöst, mit Kaliumhydroxidpulver, 30 mg (0,456 mmol), Triethylbenzylammoniumchlorid, 10 mg (0,044 mmol), und Methyliodid, 0,1 ml (1,61 mmol), bei 25°C 12 h lang umgesetzt. Anschliessend wird das Reaktionsgemisch mit Dichlormethan verdünnt und dreimal mit Wasser gewaschen. Die Dichlormethanphase wird von der Wasserphase getrennt und über Magnesiumsulfat getrocknet, und dann das organische Lösungsmittel destillativ entfernt. Anschliessend wird chromatographisch an Kieselgel/Trichlormethan und dann an Kieselgel/Dichlormethan aufgearbeitet. Ausb. 70 mg (70%) (LX) als dunkelblaugrünes Produkt, Schmp. 170-172°C,
   R_{f} (Kieselgel/Trichlormethan): 0,50.
Beispiel 12a: 1-Amino-N-(1-hexylheptyl)-perylen-3,4:9,10-tetracarbonsäure-3,4-anhydrid-9,10-imid (LXI) und 12-Amino-N-(1-hexylheptyl)-perylen-3,4:9,10-tetracarbonsäure-3,4-anhydrid-9,10-imid (LXII):
   Methode A: Eine Mischung aus (XLIII) und (XLIV), hergestellt nach Beispiel 1d, 100 mg (0,162 mmol), werden in 30 ml Tetrahydrofuran, oder einer Mischung aus Trichlormethan und Ethanol (im Gewichtsverhältnis von 3:1/Trichlormethan:Ethanol) gelöst, mit Eisen, 64 mg (1,14 mmol), und konz. Salzsäure, 0,5 ml, bei Siedetemperatur ca. 30 min lang umgesetzt. Nach dem Abkühlen wird das Reaktionsgemisch in 2N Salzsäure gegossen, und mit Aceton bis zur Lösung versetzt (bei Verwendung von Tetrahydrofuran wird kein Aceton zugesetzt). Anschliessend werden die Lösungsmittelreste abdestilliert. Der Rückstand wird filtriert. Der erhaltene Filterrückstand wird getrocknet. Man erhält (LXI) und (LXII) als dunkelblaues Pulver, das ohne Reinigung weiter umgesetzt wird.
   Methode B: Eine Mischung aus (XLIII) und (XLIV), hergestellt nach Beispiel 1d, 100 mg (0,162 mmol), Tetrahydrofuran, 30 ml, Triethylamin, 1,3 ml (9,33 mmol), werden mit Palladium/Kohle, 15 mg, bei Siedetemperatur ca. 30 min lang umgesetzt. Anschliessend wird zu dem Reaktionsgemisch Ameisensäure, 0,26 ml (6,87 mmol) und Palladium/Kohle, 30 mg, zugefügt, und bei Siedetemperatur ca. 30 min lang umgesetzt, bis sich dünnschichtchromatographisch kein (XLIII) und (XLIV) mehr nachweisen läßt. Anschliessend wird das Reaktionsgemisch auf ca. 25°C abgekühlt und vom Katalysator abfiltriert. Die weitere Aufarbeitung erfolgt wie oben beschrieben. Man erhält (LXI) und (LXII) als dunkelblaues Pulver, das ohne Reinigung weiter umgesetzt wird.
   Schmp. 292 - 294 °C, R_{f} (Kieselgel/Dichlormethan/2% Eisessig): 0,15 - 0,40.
Beispiel 12b: 1-Dimethylamino-N-(1-hexylheptyl)-perylen-3,4-anhydrid-9,10-dicarboximid (LXIII) und 12-Dimethylamino-N-(1-hexylheptyl)-perylen-3,4-anhydrid-9,10-dicarboximid (LXIV): Eine Mischung aus (LXI) und (LXII), hergestellt nach Beispiel 12a, 58 mg (0,099 mmol), Dimethylformamid, 7 ml, Ameisensäure, 2 ml, und Formalin, 1 ml (37%Gew.-%ig), werden 24h bei 105 °C umgesetzt. Anschließend wird das Reaktionsgemisch in 1N Salzsäure gegeben. Es entsteht ein blaugrüner Niederschlag, der abfiltriert wird. Anschliessend wäscht man den Niederschlag mit Wasser. Dann trocknet man den Niederschlag und arbeitet ihn unter Lichtausschluß chromatographisch an Kieselgel/Trichlormethan/Eisessig (5 Gew.-%) auf.
   Ausb. 43 mg (71%) (LXIII) und (LXIV), R_{f} (Kieselgel/Trichlormethan /Eisessig(3 Gew.-%): 0,50; die beiden Isomere werden säulenchromatographisch an Kieselgel/Trichlormethan/Eisessig (1 Gew.-%) getrennt;
   1. Fraktion: (LXIII) mit R_{f} (Kieselgel/Trichlormethan/ Eisessig (1 Gew.-%)): 0,17;
   2. Fraktion: (LXIV) mit R_{f} (Kieselgel/Trichlormethan/ Eisessig (1 Gew.-%)): 0,11.
Beispiel 13a: N,N'-Bis-(1-hexylheptyl)-benzoperylen-1',2':3,4:,9:10-hexacarbonsäure-1',2'-anhydrid-3,4:9,10-bis-(dicarboximid) (LXXI)
   N,N'-Bis(1-hexylheptyl)-perylen-3,4:9,10-bis(dicarboximid), hergestellt analog zu Chem. Ber.-121, 225-230, 1,50 g, (1,99 mmol), geschmolzenes Maleinsäureanhydrid (bei 95°C) und einige Milliliter Aceton, werden mit Chloranil, 970 mg (3,98 mmol) bei125°C 4 Tage lang erhitzt. Dann wird das Reaktionsgemisch auf ca. 30-40°C abgekühlt und in eine Mischung von Aceton und 2N Salzsäure, 250 ml, gegossen, und bei 25°C 12 h umgesetzt. Die Reaktionsmischung wird anschliessend filtriert und der Filterrückstand mehrmals mit Wasser gewaschen und dann getrocknet. Der getrocknete Filterrückstand wird dann in Trichlormethan gelöst und chromatographisch an Kieselgel/ Trichlormethan aufgearbeitet. Als erste Fraktion werden das überschüssige Chloranil und N,N'-Bis(1-hexylheptyl)-perylen-3,4:9,10-bis(dicarboximid eluiert. Anschliessend wird die Chromatographie mit einer Mischung aus Trichlormethan und Eisessig (1-5 Gew.-%) fortgesetzt und (LXXI) eluiert. Ausb. 1,20 g (71%) (LXXI) als dunkelgelbes Pulver, Schmp. > 200°C (Zers.),
   R_{f} (Kieselgel/Trichlormethan/Eisessig 10:1): 0,81.
Beispiel 13b: N,N'-Bis-(1-butylpentyl)-benzoperylen-1',2':3,4:9,10-hexacarbonsäure-1',2'-anhydrid-3,4:9,10-bis-(dicarboximid) (LXXII):
   N,N'-bis(1-Butylpentyl)-perylen-3,4:9,10-bis(dicarboximid) (hergestellt s.o.), 500 mg (0,779 mmol), geschmolzenes Maleinsäureanhydrid (bei 95°C) und einige Milliliter Aceton werden mit Chloranil, 380 mg (1,56 mmol) wie in Beispiel 14a umgesetzt und aufgearbeitet. Ausb. 450 mg (77%) (LXXII) als leuchtend orangenes Pulver, Schmp. > 300°C,
   R_{f} (Kieselgel/Trichlormethan): 0,00 - 0,12.
Beispiel 13c: N,N'-Bis-(2,5-di-*tert*-butylphenyl)-benzoperylen-1',2';3,4:9,10-hexacarbonsäure-1',2'-anhydrid-3,4:9,10-bis-(dicarboximid) (LXXIII):
   N,N'-Bis(2,5-di-*tert*.-butylphenyl)-perylen-3,4:9,10-bis(dicarboximid) (hergestellt s.o.), 300 mg (bei 95°C) und einige Milliliter Aceton werden mit Chloranil, 240 mg (0,984 mmol), wie in Beispiel 14a umgesetzt, und aufgearbeitet. Das Reaktionsgemisch wird chromatographisch an Kieselgel/Trichlormethan /Ethanol (10 Gew.-%) und dann an Kieselgel/Trichlormethan/Eisessig (10 Gew.-%) gereinigt. Ausb. 230 mg (67%) (LXXIII) als dunkelgelbes Pulver, Schmp. > 300 °C, R_{f} (Kieselgel/Trichlormethan/Ethanol (10 Gew.-%)): 0,00 - 0,16.
Beispiel 14a: N,N'-Bis-(1-hexylheptyl)-benzoperylen-1',2':3,4:9,10-hexacarbonsäure-1',2'-bis-carbethoxy-3,4:9,10-bis-(dicarboximid) (LXXIV):
   (LXXI), 110 mg (0,127 mmol), hergestellt analog Beispiel 14a, gelöst in Tetrahydrofuran, und DBU, 60 mg (0,395 mmol), werden mit Ethyliodid, 100 mg (0,641 mmol), bei 25°C 12 h lang umgesetzt. Anschliessend wird das Reaktionsgemisch in Wasser, 150 ml, gegossen, und dann mit 2N Salzsäure schwach sauer eingestellt und bei 25°C 12 h lang umgesetzt. Das Reaktionsgemisch wird filtriert, und der Filterrückstand getrocknet und anschliessend chromatographisch an Kieselgel/Trichlormethan aufgearbeitet.
   Ausb. 100 mg (85%) (LXXIV) als leuchtend oranges Pulver, Schmp.: 273-275°C, R_{f} (Kieselgel/Trichlormethan): 0,65.
Beispiel 14b: N,N'-Bis-(2,5-di-*tert*-butylphenyl)-benzopervlen-1',2':3,4:9,10-hexacarbonsäure-1',2'-bis-carbethoxy-3,4:9,10-bis-(dicarboximid) (LXXV):
   (LXXIII), 50 mg (0,060 mmol), hergestellt analog Beispiel 14c, gelöst in Tetrahydrofuran, DBU, 35 mg (0,23 mmol), werden mit Ethyliodid, 45 mg (0,29 mmol), wie in Beispiel 15a umgesetzt und aufgearbeitet. Die chromatographische Reinigung erfolgt an Kieselgel/Trichlormethan/Ethanol (3 Gew.,-%), Ausb.: 41 mg (78%) (LXXV) als dottergelbes Pulver, Schmp. > 300 °C, R_{f} (Kieselgel/Trichlormethan): 0,24.
Beispiel 15: N,N"-Bis-(1-hexylheptyl)-N'-cyclohexyl-benzoperylen-1',2':3,4:9,10-hexacarbonsäure-1',2':3,4:9,10-tris-(dicarboximid) (LXXVI):
   (LXXI), 100 mg (0,118 mmol), hergestellt analog Beispiel 14a, Cyclohexylamin, 114 mg (1,15 mmol), und Chinolin, 10 ml, werden bei 160°C 6h umgesetzt. Anschliessend wird das Reaktionsgemisch in 2N Salzsäure, 100 ml gegossen, und einige Stunden bei ca. 25 °C gerührt. Der entstandene Niederschlag wird anschliessend abfiltriert und der Filterrückstand getrocknet und chromatographisch an Kieselgel/Trichlomethan aufgearbeitet.
   Ausb.: 71 mg (66%) (LXXVI) als orangegelbes Pulver, Schmp. > 300 °C,
   R_{f} (Kieselgel/Trichlormethan): 0,63.
Beispiel 16: N,N"-Bis-(1-hexylheptyl)-N'-2,5-di-*tert*.-butylphenylbenzoperylen-1',2':3,4:9,10-hexacarbonsäure-1',2':3,4:9,10-tris-(dicarboximid) (LXXVII)
   (LXXI), hergestellt analog Beispiel 14a, 60,0 mg (0,0710 mmol), 2,5-Di-*tert*.-butylanilin, 100 mg (0,488 mmol), und DCC, 75 mg (0,364 mmol), werden mit Trifluoressigsäure (1 Tropfen) bei Siedetemperatur ca. 24 h lang gekocht. Anschliessend wird das Reaktionsgemisch mit Trichlormethan verdünnt, und dann mehrmals mit Wasser gewaschen. Die organische Lösungsmittelphase wird von der wässerigen Phase abgetrennt, im Vakuum eingeengt und chromatographisch an Kieselgel/Trichlormethan, und dann Aluminiumoxid neutral/Petrolether/3%Trichlormethan) gereinigt. Ausb.: 50 mg (70%; Ausb. 50% nach 4 Tagen Reaktionszeit, wenn nur DCC oder nur Trifluoressigsäure verwendet wird) als leuchtend-gelber Feststoff, Schmp.: 285-287°C, R_{f} (Kieselgel/Trichlormethan): 0,66.
Beispiel 17a: N,N'-Bis-(1-hexylheptyl)-benzoperylen-3,4:9,10-tetracarbonsäure-3,4:9,10-bis-(dicarboximid) (LXXVIII):
   (LXXI), hergestellt analog zu Beispiel 14a, 50 mg (0,060 mmol) und Kupfer(I)oxid, 40 mg (0,28 mmol), in Chinolin werden unter Argonatmosphäre bei 180 °C 4 h lang umgesetzt. Anschliessend wird das Reaktionsgemisch auf ca. 25-30°C abgekühlt, in 2N Salzsäure gegossen, und bei ca. 25-30°C 12 h lang behandelt. Der entstandene Niederschlag wird abgesaugt, getrocknet und chromatographisch an Kieselgel/Trichlormethan aufgearbeitet. Ausb.: 16 mg (36%) (LXXVIII) als gelbes bis orangefarbenes Pulver, Schmp. 289-291°C, R_{f} (Kieselgel/Trichlormethan): 0,82.
Beispiel 17b: N,N'-Bis-(1-hexylheptyl)-benzoperylen-3,4:9,10-tetracarbonsäure-3,4:9,10-bis-(dicarboximid) (LXXVIII):
   (LXXI), hergestellt analog Beispiel 14a, 50 mg (0,060 mmol), Kupferpulver, 40 mg (0,64 mmol) in 3-Picolin werden unter Argonatmosphäre bei Siedetemperatur 3 Tage lang umgesetzt. Anschliessend wird das Reaktionsgemisch auf ca. 25-30 °C abgekühlt und auf in 2N Salzsäure gegossen, und bei 25-30°C 12 h lang behandelt. Der entstandene Niederschlag wird abgesaugt, getrocknet und chromatographisch an Kieselgel/Trichlormethan aufgearbeitet. Ausb.: 21,3 mg (48%) (LXXVIII) als gelbes bis oranges Pulver, Schmp.: 289-291°C, R_{f} (Kieselgel/Trichlormethan): 0,82.
Beispiel 18: Zu einer Schmelze von 10 g N-Phenylmaleimid werden 0,5 g (0,66 mmol) N,N'-Bis(1-hexylheptyl)perylen-3,4:9,10-bis(dicarboximid) und 0,32 g (1,32 mmol) p-Chloranil gegeben. Diese Mischung wird mit wenig Chloroform versetzt und drei Tage auf 120°C erhitzt. Anschliessend wird mit wenig Chloroform versetzt und mit viel Methanol das Produkt ausgefällt. Nach Filtration erhält man 575 mg (94%) des gewünschten Produktes (Formel IXb, R⁴ = Phenyl, R¹=R²= 1-Hexylheptyl), R_{f}-Wert (CHCl₃) = 0,8.

## Patentansprüche

1. Kernerweiterte Perylenbisimide der allgemeinen Formeln (I) und (II) 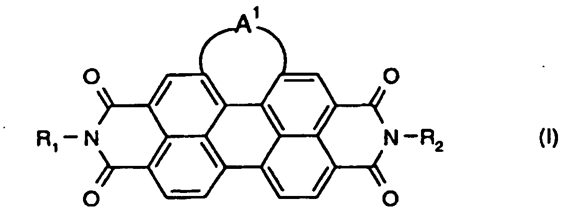 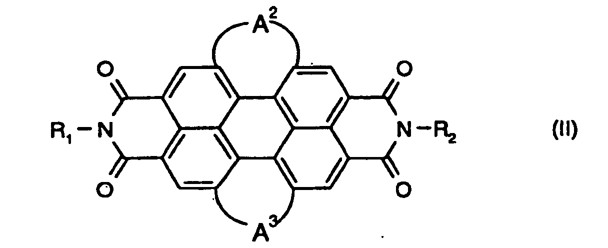 worin
R¹ und R² unabhängig voneinander für 1-(C₁-C₉Alkyl)-C₂-C₁₀Alkyl, C₃-C₂₄Cycloalkyl, oder C₆-C₁₀Aryl stehen,
A¹ und A³ unabhängig voneinander für -CH=CH- oder R³OOC-C(-)=C(-)-COOR³ stehen, und
A² für eine Verbindung der Formeln (III), (IV) oder (V) 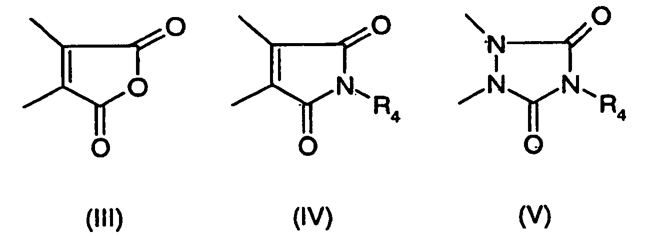 steht, werin
R³ für Wasserstoff, C₁-C₂₄Alkyl oder C₃-C₂₄Cycloalkyl, und
R⁴ für unsubstituiertes oder substituiertes C₁-C₂₄Alkyl, C₃-C₂₄Cycloalkyl, Phenyl, Benzyl, -CO-C₁-C₄Alkyl, -CO-C₆H₅ oder C₁-C₄Alkylcarbonsäure-(C₁-C₄Alkyl)-Ester stehen.

2. Verfahren zur Herstellung von Verbindungen der Formel (XXVIIa) 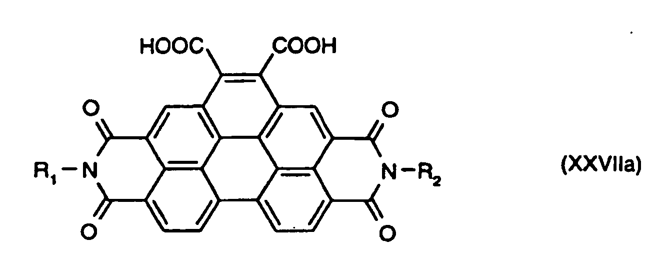 durch Hydrolyse eines Anhydrids, **dadurch gekennzeichnet, dass** man als Anhydrid eine Verbindung der Formel (VIII) 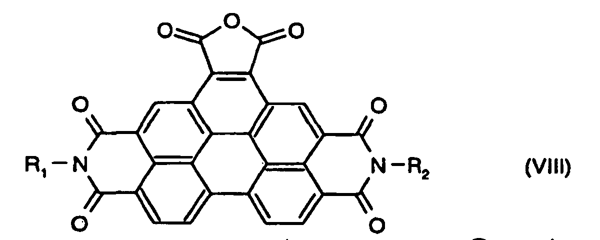 einsetzt, wobei R₁ und R₂ die in Anspruch 1 angegebene Bedeutung haben,
und diese mit einer Säure oder einer Base, bevorzugt in wässrigem Milieu, zur Reaktion bringt.

3. Verfahren zur Herstellung von Diester-Derivaten (XXVIIb) 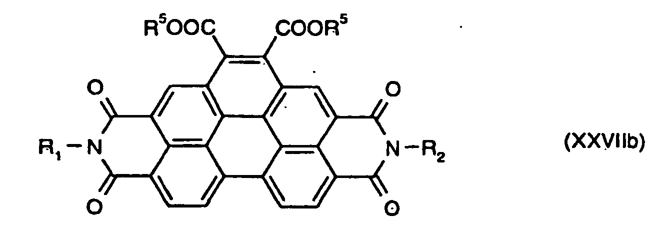 durch Veresterung einer Dicarbonsäure, **dadurch gekennzeichnet, dass** man als Dicarbonsäure eine Verbindung der Formel (XXVIIa) nach Anspruch 2 einsetzt und diese mit einem Alkohol der Formel (XXVIII), HO-R⁵ oder einem Alkylhalogenid der Formel (XXIX), Hal-R⁵, worin R⁵ für C₁-C₂₄Alkyl oder C₃-C₂₄Cycloalkyl steht, in Gegenwart einer Säure oder einer Base zur Reaktion bringt.

4. Verfahren zur Herstellung der Perylenbisimide der Formel (XI) 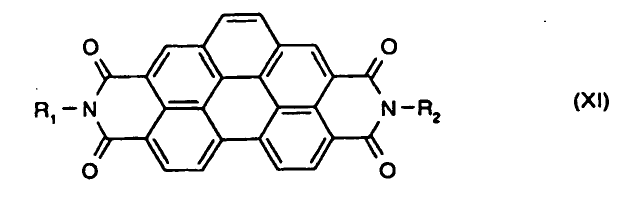 durch Decarboxylierung eines Dicarbonsäure oder eines Anhydrids mit Kupfer oder einer kupferhaltigen Verbindung, **dadurch gekennzeichnet, dass** man als Dicarbonsäure die Verbindung der Formel (XXVIIa) nach Anspruch 2 oder als Anhydrid die Verbindung (VIII) nach Anspruch 2 einsetzt und diese in Gegenwart eines Lösungsmittels zur Reaktion bringt.

5. Verwendung der erfindungsgemässen Perylene (I) oder (II) nach Anspruch 1 als Farbmittel zur Masse-Färbung von Polymeren, als Küpenfarbstoffe, als Beizenfarbstoffe, zur Herstellung von Farben, Lacken, insbesondere Automobillacken, Anstrichstoffen, Papierfarben, Druckfarben, Tinten, insbesondere zum Einsatz in Tintenstrahldruckern, für Mal- und Schreib-Zwecke, sowie in der Elektrophotographie z.B. für Trockenkopiersysteme (Xerox-Verfahren) und Laserdrucker, für Sicherheitsmarkierungs-Zwecke, als Zusatz zu Farbmitteln wie Pigmenten und Farbstoffen, bei denen eine bestimmte Farbnuance erzielt werden soll, zum Markieren von Gegenständen zum maschinellen Erkennen dieser Gegenstände über die Fluoreszenz, zur Frequenzumsetzung von Licht, zur Herstellung von passiven Anzeigeelementen für vielerlei Anzeige-, Hinweis- und Markierungszwecke, als Ausgangsmaterial für supraleitende organische Materialien, zur Feststoff-fluoreszenz-Markierung, für dekorative und künstlerische Zwecke, zu Tracer-Zwecken, als Fluoreszenzfarbstoffe in hochempfindlichen Nachweisverfahren, als Farbstoffe oder Fluoreszenzfarbstoffe in optischen Lichtsammelsystemen, in Fluoreszenz-Solar-kollektoren, in Fluoreszenzaktivierten Displays, in Kaltlichtquellen zur lichtinduzierten Polymerisation zur Darstellung von Kunststoffen, zur Materialprüfung, in Photoleitern, in fotografischen Verfahren, in Anzeige-, Beleuchtungs- oder Bildwandlersystemen, als Teil einer integrierten Halbleiterschaltung, die Farbstoffe als solche oder in Verbindung mit anderen Halbleitern enthalten, in Chemolumineszenzsystemen, in Lumineszenzimmun-assays oder anderen Lumineszenznachweisverfahren, als Signalfarben, in Farbstoff-Lasem, als optisches Speichermedium, als Rheologieverbesserer, als NIR-Farbstoffe und zur Modifizierung anorganischer Feststoffe.

## Claims

1. A nucleus-extended perylenebisimide of general formula (I) or (II) 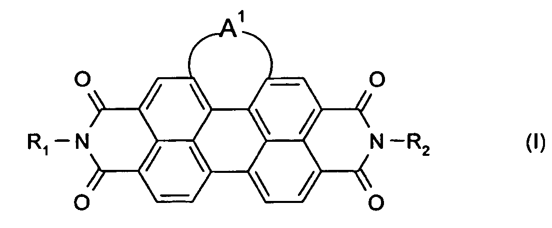 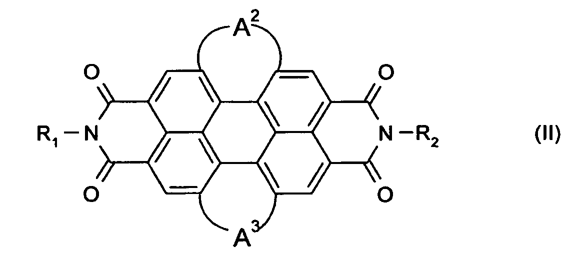 wherein
R¹ and R² are each independently of the other 1-(C₁-C₉alkyl)-C₂-C₁₀alkyl, C₃-C₂₄cycloalkyl or C₆-C₁₀aryl,
A¹ and A³ are each independently of the other -CH=CH- or R³OOC-C(-)=C(-)-COOR³, and
A² is a compound of formula (III), (IV) or (V) 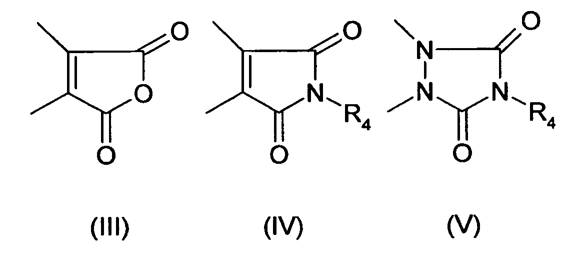 wherein
R³ is hydrogen, C₁-C₂₄alkyl or C₃-C₂₄cycloalkyl, and
R⁴ is unsubstituted or substituted C₁-C₂₄alkyl, C₃-C₂₄cycloalkyl, phenyl, benzyl, -CO-C₁-C₄alkyl, -CO-C₆H₅ or C₁-C₄alkylcarboxylic acid (C₁-C₄alkyl) ester.

2. A process for the preparation of a compound of formula (XXVIIa) 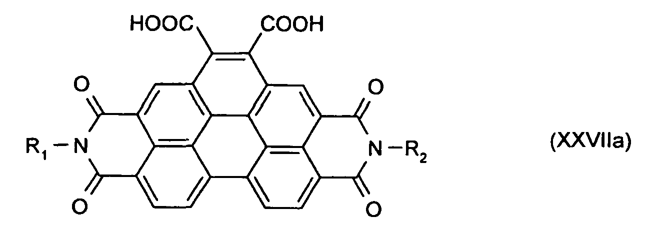 by hydrolysis of an anhydride, wherein there is used as anhydride a compound of formula (VIII) 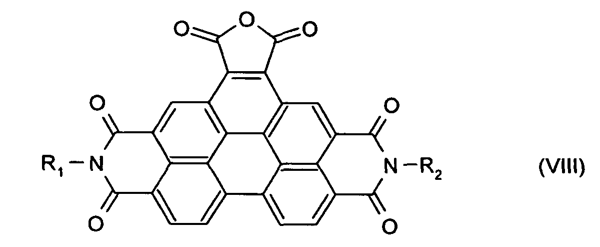 wherein R₁ and R₂ are as defined in claim 1, and that compound is reacted with an acid or a base, preferably in an aqueous medium.

3. A process for the preparation of a diester derivative (XXVIIb) 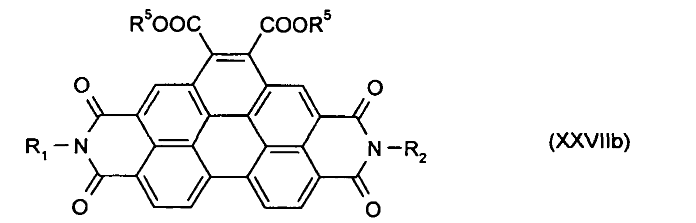 by esterification of a dicarboxylic acid, wherein a compound of formula (XXVIIa) according to claim 2 is used as dicarboxylic acid and is reacted with an alcohol of formula (XXVIII), HO-R⁵, or with an alkyl halide of formula (XXIX), Hal-R⁵, wherein R⁵ is C₁-C₂₄alkyl or C₃-C₂₄cycloalkyl, in the presence of an acid or a base.

4. A process for the preparation of a perylenebisimide of formula (XI) 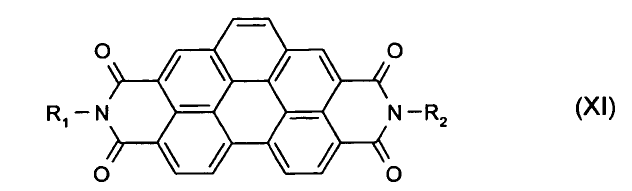 by decarboxylation of a dicarboxylic acid or an anhydride with copper or a copper-containing compound, wherein the compound of formula (XXVIIa) according to claim 2 is used as dicarboxylic acid or the compound (VIII) according to claim 2 is used as anhydride, and is reacted in the presence of a solvent.

5. Use of a perylene (I) or (II) according to claim 1 according to the invention as a colourant in the mass colouration of polymers, as a vat dye, as a mordant dye, in the manufacture of paints, lacquers, especially automotive lacquers, coating compositions, paper dyes, printing inks, inks, especially for use in ink-jet printers, for drawing and writing purposes, and in electrophotography, for example for dry-copying systems (Xerox process) and laser printers, for security-marking purposes, as an additive to colourants, such as pigments and dyes, in which a particular shade of colour is to be achieved, for labelling objects for the purpose of mechanically recognising those objects by means of fluorescence, for frequency conversion of light, for the production of passive display elements for a wide variety of display, information and labelling purposes, as starting material for supra-conducting organic materials, for fluorescent labelling of solids, for decorative and artistic purposes, for tracer purposes, as a fluorescent dye in high-sensitivity detection methods, as a dye or fluorescent dye in optical light-collecting systems, in fluorescent solar collectors, in fluorescence-activated displays, in cold-light sources for light-induced polymerisation in the preparation of plastics, for materials' testing, in photoconductors, in photographic processes, in display, illumination or image-converting systems, as part of an integrated semiconductor circuit, which contain dyes as such or in conjunction with other semiconductors, in chemoluminescent systems, in luminescent immunoassays or other luminescent detection methods, as a highlighter ink, in dye lasers, as an optical storage medium, as a rheology improver, as an NIR dye or for modifying inorganic solids.

## Revendications

1. Bisimides de pérylène à macrocycle, de formules générales (I) et (II) 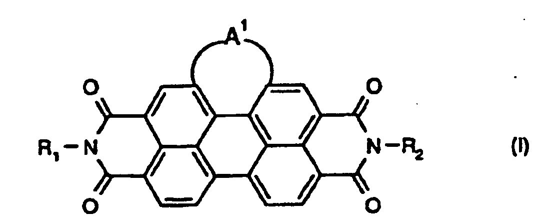 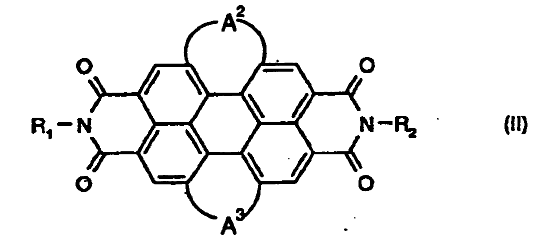 dans lesquelles
R₁ et R₂ représentent, indépendamment l'un de l'autre, un groupe 1-(alkyle en C₁ à C₉)-(alkyle en C₂ à C₁₀), cycloalkyle en C₃ à C₂₄ ou aryle en C₆ à C₁₀,
A¹ et A³ représentent, indépendamment l'un de l'autre, -CH=CH- ou R³OOC-C(-)=C(-)-COOR3, et
A² représente un composé de formule (III), (IV) ou (V) 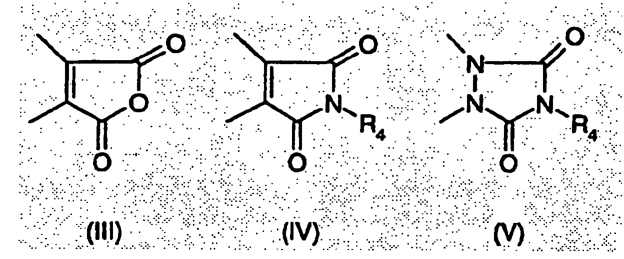
dans laquelle
R³ représente un atome d'hydrogène, un groupe alkyle en C₁ à C₂₄ ou cycloalkyle en C₃ à C₂₄ et
R⁴ représente un groupe alkyle en C₁ à C₂₄ non substitué ou substitué, cycloalkyle en C₃ à C₂₄, phényle, benzyle, -CO-(alkyle en C₁ à C₄), -CO-C₆H₅
ou ester (alkylique en C₁ à C₄) d'acide alkyl(en C₁ à C₄)carboxylique,

2. Procédé de préparation de composés de formule (XXVIIa) 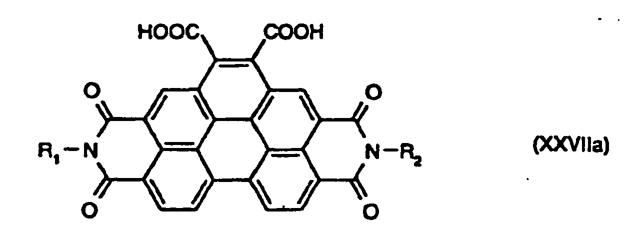 par hydrolyse d'un anhydride, **caractérisé en ce que** l'on met en oeuvre, en tant qu'anhydride, un composé de formule (VIII) 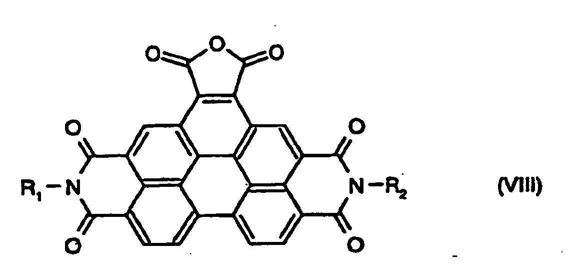 dans laquelle R₁ et R₂ ont la signification indiquée dans la revendication 1, et par réaction de celui-ci avec un acide ou avec une base, de préférence en milieu aqueux.

3. Procédé de préparation de dérivés diester (XXVIIb) 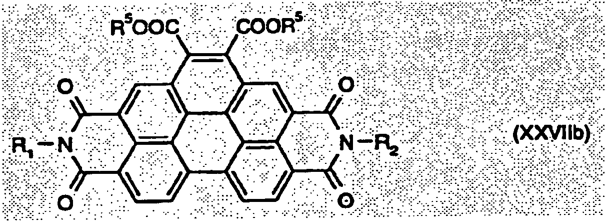 par estérification d'un acide dicarboxylique, **caractérisé en ce que** l'on met en oeuvre, en tant qu'acide dicarboxylique, un composé de formule (XXVIIa) selon la revendication 2, et par réaction de celui-ci avec un alcool de formule (XXVIII), HO-R⁵ ou un halogénure d'alkyle de formule (XXIX), Hal-R⁵, dans laquelle R⁵ représente un groupe alkyle en C₁ à C₂₄ ou cycloalkyle en C₃ à C₂₄, en présence d'un acide ou d'une base.

4. Procédé de préparation des bisimides de pérylène de formule (XI) 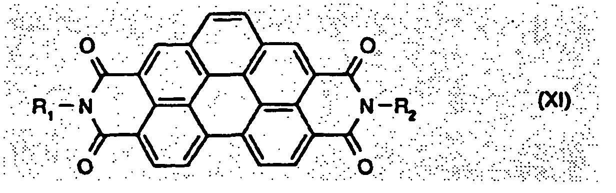 par décarboxylation d'un acide dicarboxylique ou d'un anhydride avec du cuivre ou avec un composé contenant du cuivre, **caractérisé en ce que** l'on met en oeuvre, en tant qu'acide dicarboxylique, le composé de formule (XXVIIa) selon la revendication 2 ou, en tant qu'anhydride, le composé (VIII) selon la revendication 2, et par réaction de celui-ci en présence d'un solvant.

5. Utilisation des pérylènes (I) ou (II) de la présente invention selon la revendication 1 en tant que colorants pour la coloration en masse de polymères, en tant que colorants de cuve, en tant que colorants à mordant, pour la fabrication de peintures, de vernis, en particulier de vernis pour automobiles, de peintures, de colorants pour papier, d'encres d'imprimerie, d'encres, en particulier pour des utilisations dans des imprimantes à jet d'encre, à des fins de peinture et d'écriture, ainsi que dans l'électrophotographie, par exemple pour des systèmes de copie à sec (Xérographie) et pour les imprimantes laser, pour des marquages de sûreté, en tant qu'additif de substances colorantes telles que les pigments et les colorants nécessitant une nuance de couleur déterminée, pour le marquage d'objets en vue de la reconnaissance automatique de ces objets par fluorescence, pour convertir la fréquence de la lumière, pour la fabrication d'éléments d'affichage passif à diverses fins d'affichage, d'indication et de marquage, en tant que matière première pour des matières organiques supraconductrices, pour le marquage fluorescent de solides, à des fins décoratives et artistiques, à des fins de traçage, en tant que colorants fluorescents dans des procédés de détection haute sensibilité, en tant que colorants ou colorants fluorescents dans des systèmes optiques collecteurs de lumière, dans des collecteurs solaires à fluorescence, dans des affichages activés par fluorescence, dans des sources de lumière froide pour une photopolymérisation pour la production de matières plastiques, pour les essais de matériaux, dans les photoconducteurs, dans les procédés photographiques, dans les systèmes d'affichage, d'éclairage ou de convertisseurs d'image, en tant que partie d'un circuit intégré à semi-conducteur, qui contient des colorants seuls ou conjointement avec d'autres semi-conducteurs, dans des systèmes chimioluminescents, dans des dosages immunologiques par luminescence ou d'autres procédés de détection par luminescence, en tant que colorants de signalisation, dans les lasers à colorants, en tant que support d'enregistrement optique, en tant qu'améliorants de rhéologie, en tant que colorants NIR et pour la modification de solides inorganiques.
